(19) Europäisches Patentamt
European Patent Office
Office européen des brevets

(11) **EP 3 183 383 B1**

(12) **EUROPEAN PATENT SPECIFICATION**

(45) Date of publication and mention
of the grant of the patent:
**13.03.2019 Bulletin 2019/11**

(51) Int Cl.:
*D04H 1/587* (2012.01)     *A47L 13/16* (2006.01)
*A61K 8/02* (2006.01)

(21) Application number: **15759988.7**

(22) Date of filing: **19.08.2015**

(86) International application number:
**PCT/US2015/045784**

(87) International publication number:
**WO 2016/028832 (25.02.2016 Gazette 2016/08)**

(54) **FAST DISINTEGRATING NONWOVEN BINDER**

SCHNELL ZERFALLENDES BINDEMITTEL FÜR VLIESSTOFFE

LIANT NON TISSÉ À DÉSINTÉGRATION RAPIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2014   US 201462039697 P**

(43) Date of publication of application:
**28.06.2017   Bulletin 2017/26**

(73) Proprietors:
• **Dow Global Technologies LLC
Midland, MI 48674 (US)**
• **Rohm and Haas Company
Philadelphia, PA 19106 (US)**

(72) Inventors:
• **NUNN, Maureen B.
Lower Gwynedd, PA 19002 (US)**
• **RICE, Katherine Sue
Glenside, PA 19038 (US)**
• **KLINE, Debra A.
Collegeville, PA 19426 (US)**
• **TOMLINSON, Ian A.
Midland, MI 48674 (US)**

(74) Representative: **Boult Wade Tennant LLP
Verulam Gardens
70 Gray's Inn Road
London WC1X 8BT (GB)**

(56) References cited:
**EP-A1- 2 453 049     US-A- 4 035 540
US-A- 4 117 187     US-A- 4 242 408**

**Description**

**Reference to Related Applications**

The present application claims the benefit of U.S. Provisional Application No. 62/039,697, filed August 20, 2014.

**Field of Invention**

The instant invention relates to a method for forming dispersible nonwoven substrate compositions and the dispersible nonwoven substrates made therefrom.

**Background of the Invention**

US 4,242,408 relates to easily disposable non-woven products having high wet strength at acid pH and low wet strength at base pH.

Currently, the wastewater treatment industry is focused on disposable products, such as wet wipes, which cause clogging in equipment causing difficulties and leading to downtime at wastewater treatment facilities. Therefore, wipes having adequate tensile strength while in use, but that are easily dispersible in water are desirable.

Various binders have been developed to increase dispersibility in water after toilet flushing. Examples include binders which take advantage of the differences in pH between the liquid/lotion used in the wipe and water to provide dispersibility. This way, a wipe can be intact while being used and disintegrate once being placed into the wastewater system. For example, an ion-sensitive binder can be used. These binders tend to be solvent-based and require the use of salt. However, they can fail to disperse in certain environments, such as in hard water. Other binders can be difficult to ship commercially, and must be formulated onsite. Additionally, new technology is necessary to meet changing industry standards.

Therefore, an economical dispersible nonwoven substrate composition having improved strength while also maintaining acceptable dispersibility, and emulsion polymer binders which can easily be shipped, would be desirable.

**Summary of the Invention**

The instant invention provides a method for forming a dispersible nonwoven substrate in an aqueous medium. The instant invention also provides baby wipes, personal care wipes, cleaning wipes, or any wet nonwoven having a lotion of below pH 5 or 6 made from the dispersible nonwoven substrate formed by this method.

In the first aspect of the invention there is provided a method for forming a dispersible nonwoven substrate in an aqueous medium comprising, consisting of, or consisting essentially of, contacting a fibrous substrate with an aqueous nonwoven binder comprising an emulsion polymer having a polydispersity index of at least 10, a weight average molecular weight of less than 1,000,000 and from 8 weight percent to 22 weight percent acid monomer, based on the weight of the emulsion polymer;

wherein the aqueous nonwoven binder is prepared by a method comprising the steps of: a) emulsion polymerizing at least one mono-ethylenically unsaturated monomer and at least one acid monomer in an aqueous solution using an initiator wherein the initiator is present in the aqueous solution in an amount in the range of from 0.5 weight percent to 1.5 weight percent, based on the total weight of the monomers, to form the emulsion polymer; and b) neutralizing the emulsion polymer with a neutralizer selected from the group consisting of an amine having a pKb of 4 to 7, an alkali hydroxide, and combinations thereof to form the aqueous nonwoven binder.

There is also provided a dispersible nonwoven substrate formed by the methods of any of the preceding embodiments.

In a further aspect of the invention there is provided a dispersible wipe product comprising:

a) a fibrous substrate; and

b) an aqueous nonwoven binder comprising an emulsion polymer having a polydispersity index of at least 10, a weight average molecular weight of less than 1,000,000 and from 8 weight percent to 22 weight percent acid monomer, wherein the aqueous nonwoven binder is prepared by a method comprising the steps of:

a) emulsion polymerizing at least one mono-ethylenically unsaturated monomer and at least one acid monomer in an aqueous solution using an initiator wherein the initiator is present in the aqueous solution in an amount in the range of from 0.5 weight percent to 1.5 weight percent, based on the total weight of the monomers, to form the emulsion polymer; and

b) neutralizing the emulsion polymer with a neutralizer selected from the group consisting of an amine having a pKb of 4 to7, an alkali hydroxide, and combinations thereof to form the aqueous nonwoven binder.

In further aspects of the invention there is provided a baby wipe, a personal care wipe and a cleaning wipe, each prepared with the dispersible wipe product described herein.

There are provided dispersible wipes, such as baby wipes, personal care wipes, cleaning wipes, or any wet nonwoven containing a lotion having a pH below 6, in accordance with any of the preceding embodiments.

## Detailed Description of the Invention

The instant invention provides a method for forming a dispersible nonwoven substrate in an aqueous medium and wipes such as baby, personal care, or cleaning wipes, or any wet nonwoven whose lotion is below pH 5 or 6 made from the dispersible nonwoven substrate formed by this method.

In the method for forming a dispersible nonwoven substrate in an aqueous medium of the present invention by "aqueous" herein is meant a composition in which the continuous phase is water or, in the alternative, a mixture including predominantly water but also optionally including water-miscible solvent, biocides, emoliants, buffers, chelants, and surfactants and other ingredients. By "dispersible" herein is meant that the nonwoven substrate can, under appropriate conditions, be caused to disintegrate into at least one of: smaller pieces, aggregates of fibers, individual fibers, and mixtures thereof.

By "nonwoven" herein is meant a fabric-like assembly of fibers typically in sheet or web form that is not a woven or knitted material. The nonwoven substrate includes paper; nonwoven fabrics; felts and mats; or other assemblies of fibers. The nonwoven substrate may include: cellulosic fibers such as cotton, rayon, and wood pulp; synthetic fibers such as polyester, glass, and nylon; bicomponent fibers; and mixtures thereof. In an embodiment, the nonwoven substrate has a predominant amount of fiber capable of engaging in hydrogen-bonding. In an alternative embodiment, the nonwoven substrate includes a predominant amount of cellulosic fiber. The nonwoven substrate may be formed by methods known in the art such as, for example, wet-laid, air-laid, spunbonding, spunmelt, and hydroentangling web formation. The fibers are typically selected so as their length and composition is not inimical to the ultimate dispersibility of the treated nonwoven substrate

The method for forming a dispersible nonwoven substrate in an aqueous medium comprises, consists of, or consists essentially of contacting a fibrous substrate with an aqueous nonwoven binder comprising an emulsion polymer having a polydispersity index of at least 10, a weight average molecular weight of less than 1,000,000 and from 8 weight percent to 22 weight percent acid monomer, based on the weight of the emulsion polymer.

The aqueous nonwoven binder includes an emulsion polymer; that is, a polymer prepared by the free radical polymerization of ethylenically-unsaturated monomers in an aqueous emulsion polymerization process. The emulsion polymer includes, as copolymerized units, from 8 to 22 weight percent acid monomers, based on the weight of the emulsion polymer. All ranges between 8 and 22 weight percent are included herein and disclosed herein; for example, the weight percent of acid monomers can be from a lower limit of 8, 12.5, or 14 to an upper limit of 11, 16, 20, or 22.

The emulsion polymer has a polydispersity index of at least 10. Any and all ranges greater than or equal to 10 are included herein and disclosed herein, for example, the emulsion polymer can have a polydispersity index of 15, 20, 30, 40, or 50.

The emulsion polymer also has a weight average molecular weight of less than 1,000,000. Any and all ranges less than 1,000,000 are included herein and disclosed herein, for example, the emulsion polymer can have a weight average molecular weight of less than 900,000, less than 500,000, or less than 400,000.

In various embodiments, particular ranges of acid monomer weight percent are combined with particular weight average molecular weight ranges in order to obtain the desired viscosity to produce a binder material having a solids content of at least 20 weight percent after neutralization. A composition having at least 20 weight percent solids is generally required for commercial shipping. In an embodiment, the emulsion polymer has a weight average molecular weight of less than 500,000 and from 12.5 weight percent to 20 weight percent acid monomer. In another embodiment, the emulsion polymer has a weight average molecular weight of less than 400,000 and from 14 weight percent to 16 weight percent acid monomer. In yet another embodiment, the emulsion polymer has a weight average molecular weight of less than 900,000 and from 8 weight percent to 11 weight percent acid monomer.

Acid monomers can include monoacid monomers and diacid monomers. Monoacid monomers include, for example, carboxylic acid monomers such as, for example, acrylic acid, methacrylic acid, crotonic acid, monomethyl itaconate, monomethyl fumarate, monobutyl fumarate. Examples of diacid monomers include, but are not limited to itaconic acid, fumaric acid, maleic acid; including their anhydrides, salts, and mixtures thereof.

The emulsion polymer also includes at least one other copolymerized ethylenically unsaturated monomer such as, for example, a (meth)acrylic ester monomer including methyl (meth)acrylate, ethyl (meth)acrylate, butyl (meth)acrylate, 2-ethylhexyl (meth)acrylate, decyl (meth)acrylate, hydroxyethyl (meth)acrylate, hydroxypropyl (meth)acrylate, ureido-functional (meth)acrylates and acetoacetates, acetamides or cyanoacetates of (meth)acrylic acid; styrene or substituted styrenes; vinyl toluene; butadiene; vinyl acetate or other vinyl esters; vinyl monomers such as vinyl chloride, vinylidene chloride; and (meth)acrylonitrile. The use of the term "(meth)" followed by another term such as (meth)acrylate or (meth)acrylamide as used throughout the disclosure, refers to both acrylates and methacrylates or acrylamides and methacrylamides, respectively. The at least one other copolymerized ethylenically unsaturated monomer is selected so that the emulsion polymer will have a Tg within the required range. In an embodiment, the emulsion polymer comprises ethyl acrylate, styrene, acrylic acid, and itaconic acid.

Mixtures of emulsion polymers having different compositions are also contemplated. For a mixture of two or more emulsion polymers, the acid monomer content and the Tg shall be determined from the overall composition of the emulsion polymers without regard for the number or individual composition of the emulsion polymers therein. The aqueous nonwoven binder is prepared by a method comprising the steps of: a) emulsion polymerizing at least one mono-ethylenically unsaturated monomer and at least one acid monomer in an aqueous solution using an initiator wherein the initiator is present in the aqueous solution in an amount in the range of from 0.5 weight percent to 1.5 weight percent, based on the total weight of the monomers, to form the emulsion polymer; and b) neutralizing the emulsion polymer with a neutralizer selected from the group consisting of an amine with a pKb of 4 to 7, an alkali hydroxide, and combinations thereof to form the aqueous nonwoven binder.

The emulsion polymerization techniques used to prepare the emulsion polymer are well known in the art such as, for example, as disclosed in U.S. Patents No. 4,325,856; 4,654,397; and 4,814,373. In various embodiments, the emulsion polymerization is performed at a reaction temperature of from room temperature to 100°C, depending on the initiation process (eg., thermal or redox). Conventional surfactants may be used such as, for example, anionic and/or nonionic emulsifiers such as, for example, alkali metal or ammonium alkyl sulfates, alkyl sulfonic acids, fatty acids, copolymerizable surfactants, and oxyethylated alkyl phenols. Preferred are anionic emulsifiers. The amount of surfactant used is usually 0.1% to 6% by weight, based on the weight of total monomer. Either thermal or redox initiation processes may be used. Conventional free radical initiators may be used such as, for example, hydrogen peroxide, t-butyl hydroperoxide, t-amyl hydroperoxide, ammonium and/or alkali persulfates, and water-soluble azo compounds such as azobis cyanovaleric acid, typically at a level of 0.01% to 3.0% by weight, based on the weight of total monomer. The initiator is present in a range of from 0.5% to 1.5% by weight, based on the weight of total monomer. Redox systems using the same initiators coupled with a suitable reductant such as, for example, sodium sulfoxylate formaldehyde, sodium hydrosulfite, isoascorbic acid, hydroxylamine sulfate and sodium bisulfite may be used at similar levels, optionally in combination with metal ions such as, for example iron and copper, optionally further including complexing agents for the metal. Chain transfer agents such as mercaptans may be used to lower the molecular weight of the polymers. The monomer mixture may be added neat or as an emulsion in water. The monomer mixture may be added in a single addition or in multiple additions or continuously over the reaction period using a uniform or varying composition. Additional ingredients such as, for example, free radical initiators, oxidants, reducing agents, chain transfer agents, neutralizers, surfactants, and dispersants may be added prior to, during, or subsequent to any of the stages. In various embodiments, the emulsion polymer may be prepared by a multistage emulsion polymerization process, in which at least two stages differing in composition are polymerized in sequential fashion. The molecular weight of the final polymer falls within the above molecular weight ranges. The emulsion polymer is neutralized with a neutralizer selected from the group consisting of an amine with a pKb of 4 to 7, an alkali hydroxide, and combinations thereof. Examples of alkali hydroxides that can be used as neutralizers include, but are not limited to sodium hydroxide and potassium hydroxide. Examples of amines having a pKb of 4 to 7 include, but are not limited to tromethamine, monoethanol amine, diethanol amine, and triethanol amine. In various embodiments, the neutralizer can contain from 0 to 100 weight % amine. Any and all ranges between 0 and 100 weight % are included herein and disclosed herein, for example, the neutralizer can contain 40, 50, 60, or 75 weight % amine. In various embodiments, the neutralizer can contain from 0 to 100 weight % alkali hydroxide. Any and all ranges between 0 and 100 weight % are included herein and disclosed herein, for example, the neutralizer can contain 40, 50, 60, or 75 weight % alkali hydroxide.

The calculated glass transition temperature ("Tg") of the emulsion polymer, before neutralization, is generally from -20°C to 30°C. Tgs of the polymers herein are those calculated using the Fox equation (T.G. Fox, Bull. Am. Physics Soc., Volume 1, Issue No. 3, p. 123(1956)). That is, for example, for calculating the Tg of a copolymer of monomers M1 and M2,

$$1/Tg(calc.)= w(M1)/Tg(M1) + w(M2)/Tg(M2)$$

, wherein

Tg(calc.) is the glass transition temperature calculated for the copolymer
w(M1) is the weight fraction of monomer M1 in the copolymer
w(M2) is the weight fraction of monomer M2 in the copolymer

Tg(M1) is the glass transition temperature of the homopolymer of M1
Tg(M2) is the glass transition temperature of the homopolymer of M2,
all temperatures being in °K.

The glass transition temperature of homopolymers may be found, for example, in "Polymer Handbook", edited by J. Brandrup and E.H. Immergut, Interscience Publishers. In any event the following homopolymer Tgs are to be used in the Fox equation calculation for the following polyacids: poly(methacrylic acid) Tg=185 °C; poly(acrylic acid) Tg=106 °C; poly(itaconic acid) Tg=154 °C; and poly(maleic anhydride) Tg=154 °C.

The average particle diameter of the emulsion polymer particles is typically from 30 nanometers to 500 nanometers, preferably from 200 nanometers to 400 nanometers as measured by a Brookhaven Model BI-90 Particle Sizer supplied by Brookhaven Instrument Corp., Holtsville, NY.

The aqueous nonwoven binder may include, in addition to the emulsion polymer, conventional treatment components such as, for example, emulsifiers, pigments, fillers or extenders, anti-migration aids, curing agents, coalescents, surfactants, biocides, plasticizers, organosilanes, anti-foaming agents, corrosion inhibitors, colorants, waxes, other polymers, and anti-oxidants.

In the method for forming a dispersible nonwoven substrate in an aqueous medium of the present invention the nonwoven substrate is contacted with the aqueous nonwoven binder. Typically the ratio of nonwoven binder to that of the contacted nonwoven substrate on a dry weight basis expressed as a percentage, also known as % add-on, is from 1% to 25%, preferably from 1% to 10%, selected depending on the strength of the nonwoven substrate and the desired end use. The nonwoven substrate is contacted with the aqueous nonwoven binder using conventional application techniques such as, for example, air or airless spraying, padding, saturating, roll coating, curtain coating, gravure printing, and the like. The nonwoven substrate may be contacted with the aqueous nonwoven binder so as to provide binder at or near one or both surfaces or distributed uniformly, or not, throughout the structure. It is also contemplated that the aqueous nonwoven binder may be applied in a nonuniform manner to one or both surfaces when a patterned distribution is desired.

In the method for forming a dispersible nonwoven substrate in an aqueous medium of the present invention the nonwoven substrate that has been contacted with the aqueous nonwoven binder is heated to a temperature of from 120 °C to 220 °C, preferably from 140 °C to 180 °C, for a time sufficient to achieve an acceptable level of dryness. In an embodiment, the composition is heated at a temperature and for a time sufficient to substantially dry but not to substantially cure the composition.

In the method for forming a dispersible nonwoven substrate in an aqueous medium of the present invention the contacted heated nonwoven substrate is immersed in an aqueous medium having a final pH<5, a final pH of from 3.0 to 4.99 in various embodiments, and a final pH below 3.0 in various other embodiments, to provide a dispersible nonwoven in an aqueous medium. By "final pH" herein is meant the pH (measured at 20°C) of the aqueous medium in which the contacted heated nonwoven is immersed. If the final pH<5 is not achieved with the selected nonwoven and aqueous medium and the amounts thereof, the pH is adjusted to the desired range by the addition of acidic material such as, for example, citric acid, prior to, during, or after the immersing step. The required pH of the aqueous medium is believed to contribute to a beneficial level of wet strength to the heated treated nonwoven and is also a suitable pH for certain compositions such as, for example, wipe solutions and lotions, in which the heated treated nonwoven may be stored. Typically the weight of aqueous medium is from 0.1 to 10, preferably from 0.5 to 5 times the weight of the contacted heated nonwoven substrate.

In the method for providing a dispersed nonwoven in an aqueous medium of the present invention the dispersible nonwoven of the present invention is immersed in an excess of an aqueous medium at a pH of >6.5, preferably at a final pH of >6.5; preferably at a final pH of from 6.8 to 10.0. By "an excess of an aqueous medium" is meant herein that the weight of the aqueous medium is greater than the weight of the contacted heated nonwoven. The wet strength of the heated treated nonwoven is sufficiently reduced at the designated pH (measured at 20°C) so as to facilitate its disintegration into at least one of: smaller pieces, aggregates of fibers, individual fibers, and mixtures thereof. Of course any mechanical forces that may be applied will aid in this dispersibility process, such as, for example, if the treated nonwoven were deposited in an excess of water at a pH of >6.5 and subjected to a shear force such as in a toilet flushing action.

Embodiments of the present invention can be used to make a variety of wipes, such as baby, personal care, and cleaning wipes. Other wipes having a pH of below about 5 or 6 can also be made using embodiments of the present invention.

## Examples

Abbreviations used

    AA = acrylic acid
    EA= ethyl acrylate
    IA = itaconic acid
    MMA = methyl methacrylate
    Sty = styrene

Polymer Synthesis

To a 5000ml round bottom flask fitted with a stirrer, condenser, temperature monitor, heating mantle and nitrogen inlet was added 1850 grams of deionized water which was subsequently heated to 85°C. A monomer premix was prepared from 800 grams deionized water, 26.7 grams Disponil FES 993 (a polyglycol ether sulphate sodium salt), 8.0g itaconic acid, 16.0 grams styrene, 1424 grams ethyl acrylate and 152 grams glacial acrylic acid. With the reactor water at 84°C, 7.2 grams of ammonium persulfate (APS) dissolved in 51 grams of water was added. The premix feed started at 12.1 g/min. After 20 minutes the feed rate was increased to 24. 3 g/min. At the same time, a feed of 2.4 grams ammonium persulfate dissolved in 200 grams deionized water started at 1.85 g/min. After the feeds were completed, the batch was slowly cooled over 40 minutes to about 75°C. At the targeted temperature, 7 grams of 0.15% ferrous sulfate heptahydrate aqueous solution was added. Both 2.7 grams of tBHP (70% aqueous sol of t-butyl hydroperoxide) in 50 grams of deionized water and 1.9 grams of Bruggolite FF6 (Bruggaman Chemicals) in 50 grams of deionized water were fed over one hour. The batch was cooled to room temperature and filtered. The material was subsequently neutralized with NaOH to achieve a solids of -25%. This polymer is Example 1. Other examples and comparative examples were prepared using the same process, but with varying amounts of monomers, APS, and reaction temperatures (up to about 90°C).

Whatman 4 paper was then coated with the various binders and tested for tensile strength in lotion extracted from Pure n' Gentle wipes and pH adjusted with 3% citric acid to yield a lotion having a pH of ~ 4 and in tap water having a pH of ~ 7-8. Results are shown in Tables 1 and 2, below. All polymers were neutralized 100% on a molar basis.

Table 1: Tensile Strength in Lotion on Whatman4 Filter Paper

| Sample | Polymer EA/Sty//AA/IA | Neutralizer | Mean Tensile Strength (g/2.54 cm (g/in)) | Standard Deviation |
|---|---|---|---|---|
| Comparative Example A | 89/1/9.5/.5 | Ammonia | 2149.19 | 108.7 |
| Comparative Example B | 79/1/19.5/.5 | Ammonia | 4276.05 | 272.2 |
| Comparative Example C | 89/1/9.5/.5 | Triethanol Amine | 2036.39 | 276.6 |
| Example 1 | 89/1/9.5/.5 | NaOH | 1301.01 | 106.9 |
| Example 2 | 79/1/19.5/.5 | NaOH | 894.85 | 166.2 |
| Example 3 | 89/1/9.5/.5 | Tromethamine | 740.36 | 147.6 |
| Example 4 | 79/1/19.5/.5 | Tromethamine | 917.8 | |
| Example 5 | 89/1/9.5/.5 | 40% Tromethamine/60 % NaOH | 1024.61 | 77.3 |
| Example 6 | 79/1/19.5/.5 | 40% Tromethamine/60 % NaOH | 1022.75 | 169.1 |

Table 2: Tensile Strength in Tap Water on Whatman4 Filter Paper

| Sample | Polymer EA/Sty//AA/IA | Neutralizer | Mean Tensile Strength (g/2.54 cm (g/in)) | Standard Deviation |
|---|---|---|---|---|
| Comparative Example A | 89/1/9.5/.5 | Ammonia | 2676.51 | 159.2 |
| Comparative Example B | 79/1/19.5/.5 | Ammonia | 4632.5 | 404.9 |
| Comparative Example C | 89/1/9.5/.5 | Triethanol amine | 1736.34 | 347 |
| Example 1 | 89/1/9.5/.5 | NaOH | 200.47 | 57.7 |
| Example 2 | 79/1/19.5/.5 | NaOH | 245.48 | 161.6 |
| Example 3 | 89/1/9.5/.5 | Tromethamine | 391.61 | 60.3 |
| Example 4 | 79/1/19.5/.5 | Tromethamine | 390.37 | 103.9 |
| Example 5 | 89/1/9.5/.5 | 40% Tromethamine/60% NaOH | 353.96 | 24.7 |
| Example 6 | 79/1/19.5/.5 | 40% Tromethamine/60% NaOH | 297.08 | 96.4 |

As can be seen from Tables 1 and 2, ammonia cannot be used as a neutralizer due to its volatility despite falling within the pKb range of 4 to 7.

Different polymers neutralized with various neutralizers were tested for viscosity. The results are shown in Table 3.

Table 3: Viscosity

| Example | Polymer EA/Sty//AA/IA | Neutralizer (equivalents) | % on Molar Basis | Viscosity (cps) | Viscosity 1-day (cps) |
|---|---|---|---|---|---|
| Example 7 | 89/1/9.5/.5 | Tromethamine | 100 | 310 | 128 |
| Example 8 | 89/1/9.5/.5 | NaOH | 100 | 362 | 25 |
| Example 9 | 89/1/9.5/.5 | 1.0 Tromethamine/1.0 NaOH | 200 | 150 | 20 |
| Comparative Example D | 79/1/19.5/.5 | Tromethamine | 100 | gel | gel |
| Comparative Example E | 79/1/19.5/.5 | NaOH | 100 | gel | gel |
| Example 10 | 79/1/19.5/.5 | 1.0 Tromethamine/1.0 NaOH | 200 | 4289 | 2562 |
| Example 11 | 84/1/14.5/.5 | 0.5 Tromethamine/0.5 NaOH | 100 | 370 | 370 |

As can be seen from Table 3, polymers comprising 20 weight % acid had very high viscosities.

Polymer solutions having 15 weight % acid and various molecular weights were tested for viscosity. Each polymer contained 84 wt % EA, 1 wt % Sty, 14.5 wt % AA, and 0.5 wt % IA. All polymers were neutralized with 40 equivalent% tromethamine and 60 equivalent% sodium hydroxide. The polymers were prepared at various temperatures with various amounts of APS. The amount of solids reflect the weight percent of solids in the binder solution after neutralization. Results are shown in Table 4.

Table 4: Viscosity of -25% Polymer Solution with Different Molecular Weights

| Example | Mw | Mn | PDI | Reaction Temp (°C) | APS (g) | Solubilized Viscosity (cps) | Solids of Solubilized polymer (%) |
|---|---|---|---|---|---|---|---|
| Example 12 | 350000 | 14000 | 24.7 | 84 | 1 | 1932 | 24.5 |
| Example 13 | 285000 | 13000 | 22.5 | 86 | 1 | 560 | 24.1 |
| Example 14 | 243000 | 12000 | 21 | 90 | 1 | 345 | 24.2 |
| Example 15 | 202000 | 13000 | 16 | 88 | 1.25 | 196 | 24.0 |
| Example 16 | 152000 | 11000 | 14 | 86 | 1.5 | 86 | 24.2 |
| Example 17 | 131000 | 11000 | 11.7 | 90 | 1.5 | 54 | 23.7 |

The tensile strength of a 15% acid polymer coated on Whatman4 paper was measured at various pHs. Strips of coated Whatman4 paper were cut (2.54 cm x 10.16 cm (1 in x 4 in)) and soaked in solutions having different pHs for 30 minutes. The strips were then blotted dry and were tested for strength. The polymer contained 84 wt % EA, 1 wt % Sty, 14.5 wt %

AA, and 0.5 wt % IA and was neutralized with 100% sodium hydroxide. The results are shown in Table 5.

Table 5: Tensile Strength vs. pH

| pH | Tensile (g/2.54 cm) ((g/in)) |
|---|---|
| 3.03 | 1354.032 |
| 3.95 | 1378.493 |
| 4.93 | 1136.73 |
| 6.01 | 844.746 |
| 6.93 | 422.157 |

As can be seen from Table 5, the tensile strength dropped gradually with increase in pH until a pH of almost 7 was reached.

Binders containing polymers with 84 wt % EA, 1 wt % Sty, 14.5 wt % AA, and 0.5 wt % IA with various molecular weights as a function of solids and with various amounts of solids after neutralization were produced in a 2,273 litre (500 gallon) reactor. Each binder was neutralized with 100% sodium hydroxide. The binders were tested for viscosity using a Brookfield Spindle 3 at 60 rpm. The results are shown in Tables 6-7.

Table 6 - Viscosity of Binder Having Polymers with Molecular Weight of 285000

| Solids (%) | Viscosity (cps) |
|---|---|
| 23.8 | 220 |
| 23 | 180 |
| 22 | 146 |
| 21 | 104 |
| 20 | 62 |

Table 7 - Viscosity of Binders Having Polymers with Molecular Weight of 350000

| Solids (%) | Viscosity (cps) |
|---|---|
| 20.5 | 320 |
| 19.5 | 170 |
| 19 | 135 |

(continued)

| Solids (%) | Viscosity (cps) |
|---|---|
| 18.5 | 110 |
| 18 | 98 |
| 17.5 | 89 |
| 17 | 75 |

Table 8 below shows properties of the polymers used in viscosity tests in Tables 9, 10, 11, and 12. The total solids value in Table 8 is for the polymer before neutralization. Table 9 shows the weight- and number average molecular weights of selected samples in Table 8.

Table 8: Description of Polymers used in Binders for Viscosity Tests

| Sample | Polymer EA/Sty/AA/IA | APS (wt %) | Total Solids (wt %) | Particle Size |
|---|---|---|---|---|
| Example 18 | 84/1/14.5/0.5 | 0.6 | 43.6 | 337 |
| Example 19 | 89/1/9.5/0.5 | 0.6 | 43.8 | 296 |
| Example 20 | 89/1/9.5/0.5 | 0.4 | 43.3 | 278 |
| Comparative Example F | 84/1/14.5/0.5 | 0.4 | 43.7 | 305 |
| Comparative Example G | 84/1/12/0.5 | 0.5 | 43.7 | 289 |

Table 9: Molecular Weights of Polymers

| Sample | Mw, g/mol | Mn, g/mol | Recovery (%) |
|---|---|---|---|
| Example 18 | 842,000 | 55,000 | 96.1 |
| Example 20 | 802,000 | 56,000 | 87.9 |
| Comparative Example F | 726,000 | 56,000 | 77.9 |

All binders in Tables 10-13 were 100% neutralized with triethanol amine. The viscosities were measured using a Brookfield #4 spindle. An 'error' indication means that the viscosity of the formulation was beyond the capabilities of the measuring instrument at the particular rpm. The maximum viscosities that can be measured by this spindle are 10,000 cps at 20 rpm, 4000 cps at 50 rpm, and 2000 cps at 100 rpm.

Table 10: Viscosity of Binder Formulation Containing 5% Solid Polymer

| Polymer | % Acid/% APS | pH | Viscosity after formulation | | | Viscosity after stabilizing | | |
|---|---|---|---|---|---|---|---|---|
| | | | 20 rpm | 50 rpm | 100 rpm | 20 rpm | 50 rpm | 100 rpm |
| Example 18 | 15/0.6 | 7.64 | 265.5 | 180.0 | 153.8 | 75.0 | 52.5 | 37.5 |
| Example 19 | 10/0.6 | 7.65 | 75.0 | 30.0 | 15.0 | 56.3 | 22.5 | 15.0 |
| Example 20 | 10/0.4 | 8.34 | 75.0 | 45.0 | 18.8 | 37.5 | 22.5 | 11.3 |
| Comparative Example F | 15/0.4 | | Error | Error | Error | | | |
| Comparative Example G | 12.5/0.5 | | Error | Error | Error | | | |

Table 11: Viscosity of Binder Formulation Containing 10% Solid Polymer

| Polymer | % Acid/% APS | pH | Viscosity after formulation | | | Viscosity after stabilizing | | |
|---|---|---|---|---|---|---|---|---|
| | | | 20 rpm | 50 rpm | 100 rpm | 20 rpm | 50 rpm | 100 rpm |
| Example 18 | 15/0.6 | 7.28 | 4106.0 | 2325 | 1350 | 225 | 195 | 168.8 |
| Example 19 | 10/0.6 | 7.78 | 168.8 | 135 | 90 | 131.3 | 82.5 | 60 |
| Example 20 | 10/0.4 | 7.59 | 637.0 | 390.7 | 277.5 | 112.5 | 82.5 | 67.5 |
| Comparative Example F | 15/0.4 | | Error | Error | Error | | | |
| Comparative Example G | 12.5/0.5 | | Error | Error | Error | | | |

Table 12: Viscosity of Binder Formulation Containing 15% Solid Polymer

| Polymer | % Acid/% APS | pH | Viscosity after formulation | | | Viscosity after stabilizing | | |
|---|---|---|---|---|---|---|---|---|
| | | | 20 rpm | 50 rpm | 100 rpm | 20 rpm | 50 rpm | 100 rpm |
| Example 18 | 15/0.6 | 7.81 | - | - | - | 6506 | 6247 | Error |
| Example 19 | 10/0.6 | 7.53 | 2306.0 | 1942 | 1226 | 431 | 277 | 210 |
| Example 20 | 10/0.4 | 7.52 | 4810.0 | 2857 | 1942 | 318.8 | 240 | 213.8 |
| Comparative Example F | 15/0.4 | | Error | Error | Error | | | |
| Comparative Example G | 12.5/0.5 | | Error | Error | Error | | | |

Table 13: Viscosity of Binder Formulation Containing 20% Solid Polymer

| Polymer | % Acid/% APS | pH | Viscosity after formulation | | | Viscosity after stabilizing | | |
|---|---|---|---|---|---|---|---|---|
| | | | 20 | 50 | 100 | 20 | 50 | 100 |
| Example 18 | 15/0.6 | 7.14 | Error | Error | Error | Error | Error | Error |
| Example 19 | 10/0.6 | 7.32 | Error | Error | Error | 957 | 660 | 483 |
| Example 20 | 10/0.4 | 7.45 | Error | Error | Error | 2437 | 1620 | 1181 |
| Comparative Example F | 15/0.4 | | Error | Error | Error | gel | gel | gel |
| Comparative Example G | 12.5/0.5 | | Error | Error | Error | gel | gel | gel |

As can be seen from Tables 10-13, acid levels have to be lower for formulations containing 15-20 weight % solid polymer to achieve a desirable viscosity.

### Test Methods

Test methods include the following:

### Viscosity

Viscosity was measured using a Brookfield DV-I+ Viscometer at room temperature using a Brookfield Spindle set at the specified rpm.

**Molecular Weight**

Molecular weight was measured using size-exclusion chromatography (SEC). SEC samples were prepared by bringing latex samples into THF/FA (100:5 volume/volume) at concentration of about 2 mg/mL. The prepared mixtures were shaken on a mechanical shaker overnight at ambient temperature and then sat for another 3 days. The mixtures were filtered through a 0.45 $\mu$m PTFE filter prior to SEC analysis. SEC separations were carried out on a liquid chromatograph having a HPLC pump system and a differential refractometer operated at room temperature. The SEC separation was performed in THF/FA (100:5 volume/volume) at flow rate of 1 mL/min using a SEC column set composed of two Shodex KF-806L columns (300x8 mm ID) plus Shodex KF-800D (100x8 mm ID packed with cross-linked PS-DVB gel (particle size 10 $\mu$m). The injection volume of a sample was 100 $\mu$L for the SEC separation. At a minimum, duplicate injections of each sample were performed to confirm good reproducibility of SEC separation. Commercially available polystyrene narrow standards were used to generate a calibration curve for sample molecular weight calculation.

**Tensile Strength**

Tensile strength was measured on nine 2.54 cm (1 in.) x 10.16 cm (4 in.) strips CD after a 30 min. soak in 50 g of Lotion where pH was maintained below 5 using 3% citric acid and, separately, in tap water which had been adjusted to pH=7 with 110 ppm sodium bicarbonate. To measure tensile strength, a Thwing Albert EJA tensile tester was used. It had the following settings: Gage Length - 5.08 cm (2 in), Test Speed - 30.48 (12 in/min), Break Slope Extension - 90%, and Arm Load - 0.02 kg.

The present invention may be embodied in other forms without departing from the essential attributes thereof, and, accordingly, reference should be made to the appended claims, rather than to the foregoing specification, as indicating the scope of the invention.

**Claims**

1. A method for forming a dispersible nonwoven substrate in an aqueous medium comprising:

   contacting a fibrous substrate with an aqueous nonwoven binder comprising an emulsion polymer having a polydispersity index of at least 10, a weight average molecular weight of less than 1,000,000 and from 8 weight percent to 22 weight percent acid monomer;
   wherein the aqueous nonwoven binder is prepared by a method comprising the steps of:

      a) emulsion polymerizing at least one mono-ethylenically unsaturated monomer and at least one acid monomer in an aqueous solution using an initiator wherein the initiator is present in the aqueous solution in an amount in the range of from 0.5 weight percent to 1.5 weight percent, based on the total weight of the monomers, to form the emulsion polymer; and
      b) neutralizing the emulsion polymer with a neutralizer selected from the group consisting of an amine having a pKb of 4 to7, an alkali hydroxide, and combinations thereof to form the aqueous nonwoven binder.

2. A method in accordance with claim 1 wherein the emulsion polymer has a weight average molecular weight of less than 500,000 and from 12.5 weight percent to 20 weight percent acid monomer.

3. A method in accordance with claim 1 wherein the emulsion polymer has a weight average molecular weight of less than 400,000 and from 14 weight percent to 16 weight percent acid monomer.

4. A method in accordance with claim 1 wherein the emulsion polymer has a weight average molecular weight of less than 900,000 and from 8 weight percent to 11 weight percent acid monomer.

5. A method in accordance with claim 1 further comprising
   contacting a nonwoven substrate with said aqueous nonwoven binder to form a contacted nonwoven substrate;
   heating the contacted nonwoven substrate to a temperature of from 120°C to 220°C to form a heated contacted nonwoven substrate; and
   immersing the contacted heated nonwoven substrate in an aqueous medium having a final pH of less than 5 to provide a dispersible nonwoven in an aqueous medium.

6. A method in accordance with claim 1 wherein the emulsion polymer comprises ethyl acrylate, styrene, acrylic acid, and itaconic acid.

7. A method in accordance with any one of claims 1-6 wherein the initiator is selected from the group consisting of alkali persulfates, ammonium persulfate, hydrogen peroxide, t-butyl hydroperoxide, t-amyl hydroperoxide, azobis cyanovaleric acid, and combinations thereof.

8. A method in accordance with any one of claims 1-7 wherein the neutralization step results in a polymer that is 80 % to 100 % neutralized.

9. A method in accordance with any one of claims 1-8 wherein the emulsion polymer, before neutralization in step b), has a glass transition temperature in the range of from -20°C to 30°C.

10. A dispersible wipe product comprising:

a) a fibrous substrate; and
b) an aqueous nonwoven binder comprising an emulsion polymer having a polydispersity index of at least 10, a weight average molecular weight of less than 1,000,000 and from 8 weight percent to 22 weight percent acid monomer;

wherein the aqueous nonwoven binder is prepared by a method comprising the steps of:

a) emulsion polymerizing at least one mono-ethylenically unsaturated monomer and at least one acid monomer in an aqueous solution using an initiator wherein the initiator is present in the aqueous solution in an amount in the range of from 0.5 weight percent to 1.5 weight percent, based on the total weight of the monomers, to form the emulsion polymer; and
b) neutralizing the emulsion polymer with a neutralizer selected from the group consisting of an amine having a pKb of 4 to7, an alkali hydroxide, and combinations thereof to form the aqueous nonwoven binder.

11. A baby wipe prepared with the dispersible wipe product of claim 10.

12. A personal care wipe prepared with the dispersible wipe product of claim 10.

13. A cleaning wipe prepared with the dispersible wipe product of claim 10.


**Patentansprüche**

1. Ein Verfahren zum Bilden eines dispergierbaren Vliesstoffsubstrats in einem wässrigen Medium, das Folgendes beinhaltet:

In-Kontakt-Bringen eines faserigen Substrats mit einem wässrigen Vliesstoffbindemittel, beinhaltend ein Emulsionspolymer mit einem Polydispersitätsindex von mindestens 10, einem Molekulargewicht im Gewichtsmittel von weniger als 1 000 000 und zu 8 Gewichtsprozent bis 22 Gewichtsprozent Säuremonomer; wobei das wässrige Vliesstoffbindemittel durch ein Verfahren hergestellt wird, das die folgenden Schritte beinhaltet:

a) Emulsionspolymerisieren von mindestens einem monoethylenisch ungesättigten Monomer und mindestens einem Säuremonomer in einer wässrigen Lösung unter Verwendung eines Initiators, wobei der Initiator in der wässrigen Lösung in einer Menge im Bereich von 0,5 Gewichtsprozent bis 1,5 Gewichtsprozent, bezogen auf das Gesamtgewicht der Monomere, vorliegt, um das Emulsionspolymer zu bilden; und
b) Neutralisieren des Emulsionspolymers mit einem Neutralisator, der ausgewählt ist aus der Gruppe, bestehend aus einem Amin mit einem pKb-Wert von 4 bis 7, einem Alkalihydroxid und Kombinationen davon, um das wässrige Vliesstoffbindemittel zu bilden.

2. Verfahren gemäß Anspruch 1, wobei das Emulsionspolymer ein Molekulargewicht im Gewichtsmittel von weniger als 500 000 und zu 12,5 Gewichtsprozent bis 20 Gewichtsprozent Säuremonomer aufweist.

3. Verfahren gemäß Anspruch 1, wobei das Emulsionspolymer ein Molekulargewicht im Gewichtsmittel von weniger als 400 000 und zu 14 Gewichtsprozent bis 16 Gewichtsprozent Säuremonomer aufweist.

4. Verfahren gemäß Anspruch 1, wobei das Emulsionspolymer ein Molekulargewicht im Gewichtsmittel von weniger als 900 000 und zu 8 Gewichtsprozent bis 11 Gewichtsprozent Säuremonomer aufweist.

5. Verfahren gemäß Anspruch 1, das ferner Folgendes beinhaltet:

In-Kontakt-Bringen eines Vliesstoffsubstrats mit dem wässrigen Vliesstoffbindemittel, um ein in Kontakt gebrachtes Vliesstoffsubstrat zu bilden;
Erwärmen des in Kontakt gebrachten Vliesstoffsubstrats auf eine Temperatur von 120 °C bis 220 °C, um ein erwärmtes, in Kontakt gebrachtes Vliesstoffsubstrat zu bilden; und
Eintauchen des in Kontakt gebrachten, erwärmten Vliesstoffsubstrats in ein wässriges Medium mit einem endgültigen pH-Wert von weniger als 5, um einen dispergierbaren Vliesstoff in einem wässrigen Medium bereitzustellen.

6. Verfahren gemäß Anspruch 1, wobei das Emulsionspolymer Ethylacrylat, Styrol, Acrylsäure und Itaconsäure beinhaltet.

7. Verfahren gemäß einem der Ansprüche 1-6, wobei der Initiator ausgewählt ist aus der Gruppe, bestehend aus Alkalipersulfaten, Ammoniumpersulfat, Wasserstoffperoxid, t-Butylhydroperoxid, t-Amylhydroperoxid, Azobiscyanovaleriansäure und Kombinationen davon.

8. Verfahren gemäß einem der Ansprüche 1-7, wobei der Neutralisierungsschritt in einem Polymer resultiert, das zu 80 % bis 100 % neutralisiert ist.

9. Verfahren gemäß einem der Ansprüche 1-8, wobei das Emulsionspolymer vor dem Neutralisieren in Schritt b) eine Glasübergangstemperatur im Bereich von -20 °C bis 30 °C aufweist.

10. Ein dispergierbares Wischtuchprodukt, das Folgendes beinhaltet:

a) ein faseriges Substrat; und
b) ein wässriges Vliesstoffbindemittel, beinhaltend ein Emulsionspolymer mit einem Polydispersitätsindex von mindestens 10, einem Molekulargewicht im Gewichtsmittel von weniger als 1 000 000 und zu 8 Gewichtsprozent bis 22 Gewichtsprozent Säuremonomer;
wobei das wässrige Vliesstoffbindemittel durch ein Verfahren hergestellt wird, das die folgenden Schritte beinhaltet:

a) Emulsionspolymerisieren von mindestens einem monoethylenisch ungesättigten Monomer und mindestens einem Säuremonomer in einer wässrigen Lösung unter Verwendung eines Initiators, wobei der Initiator in der wässrigen Lösung in einer Menge im Bereich von 0,5 Gewichtsprozent bis 1,5 Gewichtsprozent, bezogen auf das Gesamtgewicht der Monomere, vorliegt, um das Emulsionspolymer zu bilden; und
b) Neutralisieren des Emulsionspolymers mit einem Neutralisator, der ausgewählt ist aus der Gruppe, bestehend aus einem Amin mit einem pKb-Wert von 4 bis 7, einem Alkalihydroxid und Kombinationen davon, um das wässrige Vliesstoffbindemittel zu bilden.

11. Ein Babywischtuch, hergestellt mit dem dispergierbaren Wischtuchprodukt gemäß Anspruch 10.

12. Ein Körperpflegewischtuch, hergestellt mit dem dispergierbaren Wischtuchprodukt gemäß Anspruch 10.

13. Ein Reinigungswischtuch, hergestellt mit dem dispergierbaren Wischtuchprodukt gemäß Anspruch 10.


**Revendications**

1. Un procédé pour former un substrat non tissé dispersable dans un milieu aqueux comprenant :

la mise en contact d'un substrat fibreux avec un liant non tissé aqueux comprenant un polymère en émulsion ayant un indice de polydispersité d'au moins 10, une masse moléculaire moyenne en poids inférieure à 1 000 000 et de 8 pour cent en poids à 22 pour cent en poids de monomère acide ;
dans lequel le liant non tissé aqueux est préparé par un procédé comprenant les étapes de :

a) polymérisation en émulsion d'au moins un monomère à insaturation mono-éthylénique et d'au moins un monomère acide dans une solution aqueuse à l'aide d'un initiateur dans lequel l'initiateur est présent dans la solution aqueuse dans une quantité comprise dans la plage allant de 0,5 pour cent en poids à 1,5 pour cent en poids, rapporté au poids total des monomères, afin de former le polymère en émulsion ; et
b) neutralisation du polymère en émulsion avec un agent de neutralisation sélectionné dans le groupe constitué d'une amine ayant un pKb de 4 à 7, d'un hydroxyde alcalin, et de combinaisons de ceux-ci afin de former le liant non tissé aqueux.

2. Un procédé selon la revendication 1 dans lequel le polymère en émulsion a une masse moléculaire moyenne en poids inférieure à 500 000 et allant de 12,5 pour cent en poids à 20 pour cent en poids de monomère acide.

3. Un procédé selon la revendication 1 dans lequel le polymère en émulsion a une masse moléculaire moyenne en poids inférieure à 400 000 et allant de 14 pour cent en poids à 16 pour cent en poids de monomère acide.

4. Un procédé selon la revendication 1 dans lequel le polymère en émulsion a une masse moléculaire moyenne en poids inférieure à 900 000 et allant de 8 pour cent en poids à 11 pour cent en poids de monomère acide.

5. Un procédé selon la revendication 1 comprenant en outre
la mise en contact d'un substrat non tissé avec ledit liant non tissé aqueux afin de former un substrat non tissé mis en contact ;
le chauffage du substrat non tissé mis en contact jusqu'à une température allant de 120 °C à 220 °C afin de former un substrat non tissé mis en contact chauffé ; et l'immersion du substrat non tissé chauffé mis en contact dans un milieu aqueux ayant un pH final inférieur à 5 afin de fournir un non-tissé dispersable dans un milieu aqueux.

6. Un procédé selon la revendication 1 dans lequel le polymère en émulsion comprend de l'acrylate d'éthyle, du styrène, de l'acide acrylique, et de l'acide itaconique.

7. Un procédé selon l'une quelconque des revendications 1 à 6 dans lequel l'initiateur est sélectionné dans le groupe constitué de persulfates alcalins, du persulfate d'ammonium, du peroxyde d'hydrogène, de l'hydroperoxyde de t-butyle, de l'hydroperoxyde de t-amyle, de l'acide azobis cyanovalérique, et de combinaisons de ceux-ci.

8. Un procédé selon l'une quelconque des revendications 1 à 7 dans lequel l'étape de neutralisation conduit à un polymère qui est neutralisé à hauteur de 80 % à 100 %.

9. Un procédé selon l'une quelconque des revendications 1 à 8 dans lequel le polymère en émulsion, avant la neutralisation à l'étape b), a une température de transition vitreuse comprise dans la plage allant de -20 °C à 30 °C.

10. Un produit de type lingette dispersable comprenant :

a) un substrat fibreux ; et
b) un liant non tissé aqueux comprenant un polymère en émulsion ayant un indice de polydispersité d'au moins 10, une masse moléculaire moyenne en poids inférieure à 1 000 000 et allant de 8 pour cent en poids à 22 pour cent en poids de monomère acide ;
dans lequel le liant non tissé aqueux est préparé par un procédé comprenant les étapes de :

a) polymérisation en émulsion d'au moins un monomère à insaturation mono-éthylénique et d'au moins un monomère acide dans une solution aqueuse à l'aide d'un initiateur dans lequel l'initiateur est présent dans la solution aqueuse dans une quantité comprise dans la plage allant de 0,5 pour cent en poids à 1,5 pour cent en poids, rapporté au poids total des monomères, afin de former le polymère en émulsion ; et
b) neutralisation du polymère en émulsion avec un agent de neutralisation sélectionné dans le groupe constitué d'une amine ayant un pKb de 4 à 7, d'un hydroxyde alcalin, et de combinaisons de ceux-ci afin de former le liant non tissé aqueux.

11. Une lingette pour bébé préparée avec le produit de type lingette dispersable de la revendication 10.

**12.** Une lingette pour soins personnels préparée avec le produit de type lingette dispersable de la revendication 10.

**13.** Une lingette nettoyante préparée avec le produit de type lingette dispersable de la revendication 10.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62039697 A
- US 4242408 A
- US 4325856 A
- US 4654397 A
- US 4814373 A

**Non-patent literature cited in the description**

- **T.G. FOX.** *Bull. Am. Physics Soc.,* 1956, vol. 1 (3), 123
- Polymer Handbook. Interscience Publishers